# EUROPEAN PATENT SPECIFICATION

(11) **EP 1 567 869 B1**
(45) Date of publication and mention of the grant of the patent: **22.08.2007**
(21) Application number: 03796034.1
(22) Date of filing: 21.11.2003
(51) Int. Cl.: G01N 33/74, G01N 33/94

(54) **METHOD FOR SCREENING FOR COMPOUNDS AS POTENTIAL SEDATIVES OR ANXIOLYTICS**
VERFAHREN ZUM SCREENING FÜR VERBINDUNGEN MIT SEDATIVER ODER ANXIOLYTISCHER POTENZ
PROCEDE DESTINE A ANALYSER UN COMPOSE CHIMIQUE POUR EN RECHERCHER LE POTENTIEL EN TANT QUE SEDATIF OU QU'ANXIOLYTIQUE

(30) Priority: 28.11.2002 DK 200201840
(43) Date of publication of application: 31.08.2005
(73) Proprietor: NEUROSEARCH A/S, 2750 Ballerup (DK)
(72) Inventor: Mikkelsen, Jens Damsgaard, NeuroSearch A/S, 2750 Ballerup (DK)
(74) Representative: Abildgren, Michael Padkjaer
(86) International application number: PCT/EP2003/050860
(87) International publication number: WO 2004/048980

(56) References cited:
- WO-A-00/44752
- WO-A-01/05222
- WO-A-02/40700
- CRESTANI FLORENCE ET AL: "Mechanism of action of the hypnotic zolpidem in vivo" BRITISH JOURNAL OF PHARMACOLOGY, vol. 131, no. 7, December 2000 (2000-12), pages 1251-1254, XP001189786 ISSN: 0007-1188
- MCKERNAN R M ET AL: "Sedative but not anxiolytic properties of benzodiazepines are mediated by the GABAA receptor alpha1 subtype" NATURE NEUROSCIENCE, vol. 3, no. 6, June 2000 (2000-06), pages 587-592, XP002277202 ISSN: 1097-6256 cited in the application
- KRALIC J E ET AL: "DELETION OF GABAA RECEPTOR ( GABAA - R ) alpha1 SUBUNIT ALTERS BENZODIAZEPINE ( BZD ) SITE PHARMACOLOGY, FUNCTION AND RELATED BEHAVIOR." SOCIETY FOR NEUROSCIENCE ABSTRACT VIEWER AND ITINERARY PLANNER, vol. 2002, 2002, page Abstract No. 39.12 XP001189785 32nd Annual Meeting of the Society for Neuroscience;Orlando, Florida, USA; November 02-07, 2002
- STAHL STEPHEN M: "Selective actions on sleep or anxiety by exploiting GABA-A/benzodiazepine receptor subtypes" JOURNAL OF CLINICAL PSYCHIATRY, vol. 63, no. 3, March 2002 (2002-03), pages 179-180, XP008029821 ISSN: 0160-6689
- BARBACCIA M L ET AL: "Stress and neurosteroids in adult and aged rats." EXPERIMENTAL GERONTOLOGY. ENGLAND 1998 NOV-DEC, vol. 33, no. 7-8, November 1998 (1998-11), pages 697-712, XP001189793 ISSN: 0531-5565
- CULLINAN WILLIAM E: "GABAA receptor subunit expression within hypophysiotropic CRH neurons: A dual hybridization histochemical study" JOURNAL OF COMPARATIVE NEUROLOGY, vol. 419, no. 3, 10 April 2000 (2000-04-10), pages 344-351, XP008029812 ISSN: 0021-9967

## Description

The present invention relates to a method for screening a chemical compound for its potential as a sedative or anxiolytica.

### BACKGROUND ART

GABA is the major inhibitory neurotransmitter in the mammalian brain and the GABA_{A} receptor is the site of action of benzodiazepines. Multiple isoforms of GABA_{A} receptor exist; each receptor comprises a pentameric complex formed by co-assembly of subunits selected from 16 genes (α₁₋₆, β₁₋₃, γ₁₋₃, δ, ε, Π, and θ) creating a chloride ion-channel.

The most abundant GABA_{A} receptor in the mammalian brain comprises α, β, and γ subunits, and the classical anxiolytic benzodiazepines bind to these receptors if they contain α_{1,2,3 or 5} and γ₂ subunits. Because the subtypes are differently expressed in the brain as well as in other organs and because different subtypes are considered to be involved in different function, subtype specific compounds have been developed both with agonistic, antagonistic and inverse agonistic potentials. An example of such a subtype specific compound is the non-anxiolytic imidazopyridine zolpidem, which is highly selective for α₁ containing GABA_{A} receptors and is used as a short acting sedative in humans. α₂, α₃, and α₅ benzodiazepines sites are considered to be involved in anxiolytic properties and similar attempts have been made develop specific compounds for these sites. Such an example is the compound L-838,417, which is a selective α₂, α₃, and α₅ agonist [McKernan et al., Nat. Neurosci. 2000 June; 3(6); 587-92].

In order to develop new subtype specific compounds and to assess their efficacy *in vivo,* it is necessary to test new chemical entities (NCE's) in living animals. As the site of action is in the brain, behavioural testing is essential to determine pharmacokinetic and other ADME properties of the NCE. Furthermore, it is essential to determine the efficacy in terms of hypnotic, sedative, anxiolytic, muscle relaxant, and anticonvulsive properties. Behavioural analyses in animals involve a number of so-called anxiety models, which detect the subjects' capability to take risks. The major problem with these models is that they are only partly predictive to assess a full behavioural response to a NCE with *in vitro* effect on the GABA_{A} receptor. There exists no *in vivo* prediction of alpha selectivity. Furthermore, because some of these compounds are sedative, it is hard to determine if their lack of action is specific or linked to its sedative properties. A method that activates systems in the brain relevant for the action of subtype specificity of NCE is therefore badly needed.

The hypothalamo-pituitary-adrenal (HPA) axis consists of the hypothalamic corticotrophin releasing factor (CRF) neurons in the medial parvocellular nuclei of the paraventricular nucleus (PVN), the corticotrophs of the anterior pituitary, and the steroid-producing cells in the adrenal cortex. The HPA axis drives the release of circulating corticosteroids in the blood, and is thus a central component of the stress response. The HPA axis is under negative feedback, as increasing concentrations of plasma corticosteroids will inhibit the activity of the HPA axis via specific receptors for glucocorticosteroids. The HPA axis is under influence by other centers in the brain, and thereby it is activated in response to anxiety and fear. Pharmacological intervention can affect either directly on stress-related pathways, on the CRF neurons, or peripherally to affect the inhibitory feedback on the axis.

Diazepam has been shown to slightly stimulate the HPA axis at the level of the hypothalamic corticotrophin releasing factor (CRF) neurons.

WO 01/05222 and WO 02/40700 relate to transgenic mice deficient in corticotrophin releasing factor receptor 2.

Crestani, F. et al, British Journal of Pharmacology, vol 131 (7), 1251-1254 describe the mechanism of action of the hypnotic zolpidem *in vivo.*

McKernan, R. M. et al, Nature Neuroscience, vol 3 (6), 587-592 describe that sedative but not anxiolytic properties of benzodiazepines are mediated by the GABA_{A} receptor α₁ subtype.

WO 00/44752 relates to triazolo-pyridazine derivatives as ligands for GABA receptors.

### SUMMARY OF THE INVENTION

According to the invention it has now been found that activation of the HPA axis is coupled to mediation through the GABA_{A} receptors comprising α₁-subtypes and thereby coupled to a sedative effect of the compound.

Thus, in a first aspect, the invention relates to a method for screening a GABA_{A} receptor modulator for its potential as a sedative or anxiolytica.

Other objects of the invention will be apparent to the person skilled in the art from the following detailed description and examples.

### DETAILED DISCLOSURE OF THE INVENTION

In a first aspect, the invention provides a method for screening a GABA_{A} receptor modulator for its potential as a sedative or anxiolytica, which method comprises the following steps:
a) exposing the compound to a non-human test animal whereby the compound is to be administered; and
b) measuring the effect of the compound on the activity of the HPA axis; and
c) selecting the compound as a sedative drug candidate if the compound stimulates the HPA axis, or
   selecting the compound as an anxiolytica drug candidate if the compound has no effect on the HPA axis.

In one embodiment, the test animal is a non-human animal, such as a mammal. In a further embodiment, the test animal is a rodent, such as a mouse or a rat. In a still further embodiment, the test animal is a non-mammalian vertebrate, such as a reptile, bird or fish.

In a further embodiment, the route of exposure of the compound is intraperitoneal (i.p.), intraveneous (i.v.), peroral (p.o.) or subcutaneous (s.c.).

ln a still further embodiment, the measurement of the activity of the HPA axis is performed by measuring, in a blood sample from the test animal after administration, the level of plasma corticosterone and/or ACTH.

In a further embodiment, the method for screening comprises the further step of: c1) selecting the compound as a sedative drug candidate if the compound stimulates the HPA axis. In a special embodiment, the stimulation of the HPA axis is at least a 2-fold increase, preferably at least a 3-fold increase, in corticosterone and/or ACTH over vehicle within the first two hours of administration.

In a still further embodiment, the method for screening comprises the further step of: c2) selecting the compound as an anxiolytica drug candidate if the compound has no effect on the HPA axis. In a special embodiment, no effect on the HPA axis is less than a 50 percent increase, preferably less than a 25 percent increase, in corticosterone and/or ACTH over vehicle within the first two hours of administration.

### Measurement of HPA axis activity

A good measure of the activity of the HPA axis (hypothalamus-pituitary-adrenal axis) is a measure of those hormones that are released in response to the activation, i.e. the adrenocorticotrophic hormone (ACTH) and glucocorticoids (such as corticosterone or cortisol). These hormones can easily be measured in the blood, urine and the saliva of the test animal. Furthermore, activation of the CRF neurons in the hypothalamus can be assessed as activity of transcriptional activation in the neurons (Hoffman et al., J Neuroendocriol. 2002 Apr.; 14(4); 259-68).

One example of measuring the activity of the HPA axis is as follows: The animal is treated with the NCE and sacrificed within an hour. As the release of ACTH occurs within an hour after the stimulation of the CRF neurons, animals are sacrificed at t=0, 5, 15, 30 and 60 minutes after administration. Trunk blood is taken, serum separated and levels of ACTH is measured in the serum by specific radioimmunoassay. Similarly, the level of glucocorticosteroids are determined at t=0, 30, 60, and 120 minutes (the response occurs somewhat later than ACTH) using a radioimmunoassay.

### Pharmaceutical Compositions

While a GABA_{A} receptor modulator as identified by the method according to the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, pharmaceutical compositions comprising the GABA_{A} receptor modulator of the invention, or a pharmaceutically acceptable salt or derivative thereof, together with one or more pharmaceutically acceptable carriers therefor, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition may be administered by any convenient route which suit the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition may be prepared by the skilled person using standard and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

### BRIEF DESCRIPTION OF THE DRAWING

The present invention is further illustrated by reference to the accompanying drawing, in which:
Fig. 1 shows the effect of increasing doses of zolpidem and L-838,417 on plasma corticosterone levels in mice. The data represent mean ± S.E.M. of 5 mice per group. Significant effect of compound compared to vehicle *p<0.05.

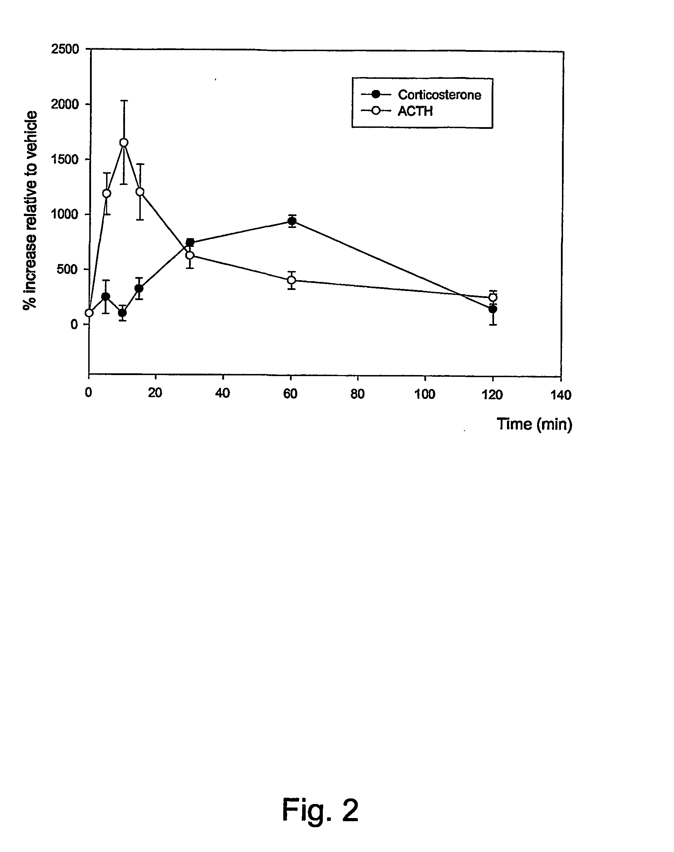

Fig. 2 shows the time course of the effect of 10 mg/kg zolpidem on the HPA axis. The rise in plasma ACTH precedes the rise in corticosterone.

The following example will illustrate the invention further, however, it is not to be construed as limiting.

### EXAMPLES

### Example 1

### Measuring the affect on the HPA axis of Zolpidem in mice

Adult male NMRI mice (23-27 g.) were purchased from Møllegaarden (Denmark). The animals were received at the animal facility, and housed 5 per cage under 12:12 light: dark cycle, humidity and temperature controlled room for at least 7 days before the experiment. Food and water were available ad libitum. All procedures were conducted in accordance with the Danish National Guide for Care and Use of Laboratory animals. Zolpidem was purchased from Tocris Ltd (Bristol, UK) and L-838,417 synthesised according to WO 98/04559 and was injected in a volume of 10 ml/kg and dissolved in 5% Chremophor.

The two drugs were administered (i.p.) at doses 0,025, 1,25, 2.5, 12.5 and 25 mg/kg. The mice were returned to their home cages and sacrificed by decapitation 60 minutes after drug administration and trunk blood was collected in centrifuge tubes containing 2 mg EDTA. Plasma aliquots were stored at -20°C until hormone levels were determined.

Plasma corticosterone was measured directly without prior extraction by a commercially [¹²⁵I] corticosterone radioimmunoassay kit from Amersham. The experiment was performed twice. The data were analysed by a two-way analysis of variance (ANOVA) followed by the Dunn's test. All data are represented as group means and the standard error of means (SEM).

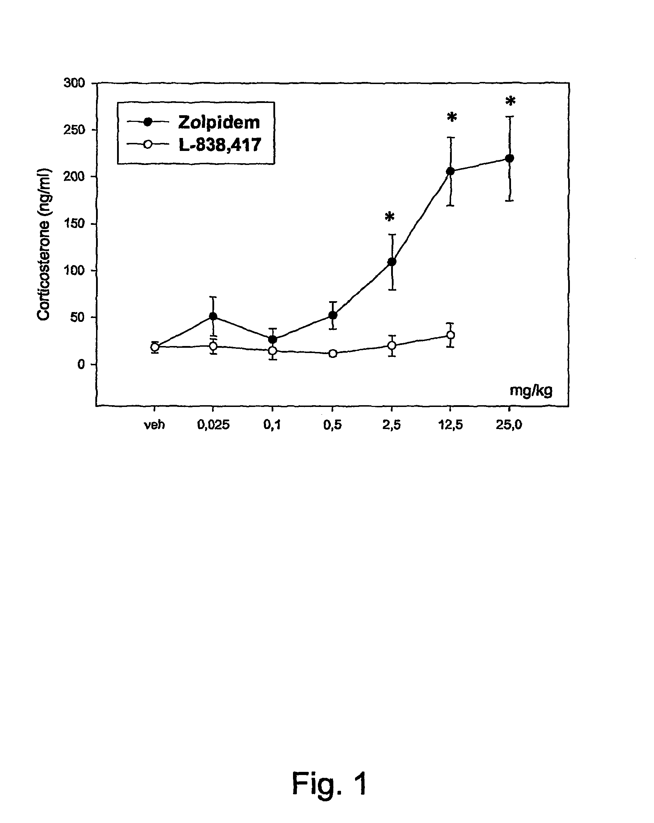

Zolpidem significantly and dose-dependently increased plasma corticosterone in doses from 0,5 mg/kg. As demonstrated in Fig. 1 the effect reached a maximum at 12.5 mg/kg and not further increased by 25 mg/kg. In contrast, L-838,417 had no effect on corticosterone in doses up to 12.5 mg/kg (Fig. 1). When tested 2 h after administration of 12.5 mg/kg L-838,417 no effects on plasma corticosterone was observed.

## Claims

1. A method for screening a GABA_{A} receptor modulator for its potential as a sedative or anxiolytica, which method comprises the following steps:
a) exposing the compound to a non-human test animal whereby the compound is to be administered; and
b) measuring the effect of the compound on the activity of the HPA axis; and
c) selecting the compound as a sedative drug candidate if the compound stimulates the HPA axis, or
selecting the compound as an anxiolytica drug candidate if the compound has no effect on the HPA axis.

2. The method according to claim 1, wherein the test animal is a mouse or a rat.

3. The method according to claim 2, wherein the measurement of the activity of the HPA axis is performed by measuring, in a blood sample from the test animal after administration, the level of plasma corticosterone and/or ACTH.

4. The method according to any one of claims 1-3, comprising the step:
c1) selecting the compound as a sedative drug candidate if the compound stimulates the HPA axis.

5. The method according to any one of claims 1-3, comprising the step:
c2) selecting the compound as an anxiolytica drug candidate if the compound has no effect on the HPA axis.

## Patentansprüche

1. Verfahren zum Screening eines GABA_{A}-Rezeptor-Modulators auf sein Potential als Sedativum oder Anxiolytikum, wobei das Verfahren die folgenden Schritte umfasst:
a) Einwirkenlassen der Verbindung auf ein nichtmenschliches Versuchstier, wobei die Verbindung zu verabreichen ist; und
b) Messen der Wirkung der Verbindung auf die Aktivität der HPA-Achse; und
c) Auswählen der Verbindung als Arzneistoffkandidat für ein Sedativum, wenn die Verbindung die HPA-Achse stimuliert, oder
Auswählen der Verbindung als Arzneistoffkandidat für ein Anxiolytikum, wenn die Verbindung keine Wirkung auf die HPA-Achse hat.

2. Verfahren nach Anspruch 1, wobei das Versuchstier eine Maus oder eine Ratte ist.

3. Verfahren nach Anspruch 2, wobei die Messung der Aktivität der HPA-Achse durchgeführt wird, indem nach der Verabreichung der Plasmaspiegel von Corticosteron und/oder ACTH in einer Blutprobe von dem Versuchstier gemessen wird.

4. Verfahren nach einem der Ansprüche 1 bis 3, umfassend den Schritt:
c1) Auswählen der Verbindung als ein Arzneistoffkandidat für ein Sedativum, wenn die Verbindung die HPA-Achse stimuliert.

5. Verfahren nach einem der Ansprüche 1 bis 3, umfassend den Schritt:
c2) Auswählen der Verbindung als Arzneistoffkandidat für ein Anxiolytikum, wenn die Verbindung keine Wirkung auf die HPA-Achse hat.

## Revendications

1. Procédé de criblage d'un modulateur du récepteur GABA_{A} pour son potentiel en tant que sédatif ou qu'anxiolytique, ce procédé comprenant les étapes suivantes :
a) exposition du composé à un animal de laboratoire non humain auquel le composé doit être administré ; et
b) mesure de l'effet du composé sur l'activité de l'axe HPA ; et
c) sélection du composé en tant que substance médicamenteuse sédative candidate si le composé stimule l'axe HPA, ou
sélection du composé en tant que substance médicamenteuse anxiolytique candidate si le composé n'a pas d'effet sur l'axe HPA.

2. Procédé selon la revendication 1, dans lequel l'animal de laboratoire est une souris ou un rat.

3. Procédé selon la revendication 2, dans lequel la mesure de l'activité de l'axe HPA est effectuée en mesurant, dans un échantillon sanguin de l'animal de laboratoire après administration, le taux de cortico-stérone et/ou d'ACTH plasmatique.

4. Procédé selon l'une quelconque des revendications 1 à 3, comprenant l'étape :
c1) sélection du composé en tant que substance médicamenteuse sédative candidate si le composé stimule l'axe HPA.

5. Procédé selon l'une quelconque des revendications 1 à 3, comprenant l'étape :
c2) sélection du composé en tant que substance médicamenteuse anxiolytique candidate si le composé n'a pas d'effet sur l'axe HPA.
